# EUROPEAN PATENT APPLICATION

(11) **EP 1 455 364 A1**
(43) Date of publication of application: **08.09.2004**
(21) Application number: 03100558.0
(22) Date of filing: 06.03.2003
(51) Int. Cl.: G21G 1/10, H05H 6/00, G21G 4/08

(54) **Method for producing actinium-225**

(71) Applicant: EUROPEAN COMMUNITY, 1049 Brussels (BE)
(72) Inventor: Abbas, Kamel, 21023 Besozzo (IT); Apostolidis, Christos, 69115 Heidelberg (DE); Janssens, Willem, 76694 Forst (DE); Stamm, Hermann, 21020 Castelveccana (IT); Nikula, Tuomo, 76297 Stutensee (DE); Carlos Marquez, Ramon, 76227 Karlsruhe-Durlach (DE)
(74) Representative: Kihn, Pierre Emile Joseph

(57) **Abstract**

A method for producing Ac-225 is presented, wherein Ac-225 is produced by bombardment of Ra-226 with deuterons accelerated in a cyclotron. The deuterons preferably have an incident energy of between 15 and 22 MeV. The method, which allows production of Ac-225 at high yields and purity levels, is particularly interesting in view of Bi-213 generation.

## Description

### FIELD OF THE INVENTION

The present invention generally relates to a method for producing Acti n-ium-225.

### BACKGROUND OF THE INVENTION

Production of Actinium-225 (Ac-225) and its daughter Bismuth-213 (Bi-213) is of great interest for cancer therapy, as they constitute preferred radionuclides for alpha-immunotherapy purposes. Indeed, to selectively irradiate cancer cells, alpha-immunotherapy uses alpha-emitters such as Bi-213 and possibly Ac-225 that are linked, through a bifunctional chelator, to monoclonal antibodies or peptides.

EP-A-0 962 942 discloses a method for producing Ac-225, which consists in irradiating a target containing Ra-226 with protons in a cyclotron, so that metastable radionuclei are transformed into Actinium by emitting neutrons. It is to be noted that this method allows to obtain the desired Ac-225, but also considerable quantities of other highly undesired radionuclides, especially Ac-224 and Ac-226. In order to eliminate these undesired radionuclides, the post-irradiation process is delayed since Ac-224 and Ac-226 present a relatively short half-life compared with Ac-225 (half-life 10 days). This waiting period however also leads to a considerable loss of Ac-225.

In order to increase the yield of Ac-225, EP-A-0 962 942 proposes to irradiate a target of Ra-226 with protons having an incident energy of between 10 and 20 MeV, preferably of about 15 MeV.

### OBJECT OF THE INVENTION

The object of the present invention is to provide an improved method of producing Ac-225, which provides higher yield of Ac-225. This object is achieved by a method as claimed in claim 1.

### SUMMARY OF THE INVENTION

According to the present invention, Actimium-225 is produced by irradiating a target of Radium-226 with deuterons. The present method is based on the transformation of instable Ra-226 radionuclei -due to deuteron bombardment-into Ac-225 by emitting neutrons (this reaction is hereinafter noted Ra-226(d,3n)Ac-225). It has been found that reaction Ra-226(d,3n)Ac-225 has a higher cross-section -i.e. has a greater likelihood to occur- than the conventionally used reaction involving proton bombardment of Ra-226. It follows that the method of the invention permits production of Ac-225 at higher yields than the conventional method. It is further to be noted that the present method also allows production of Ac-225 at high purity levels, which is important for therapeutic use. The present method is thus particularly well adapted for producing Ac-225 in view of Bi-213 generation.

The deuterons are preferably bombarded on the target of Ra-226 with an incident energy of between 15 and 22 MeV, at which Ac-225 can be produced with high yields and purity levels. A more preferred energy range for the deuterons is between 18 and 20 MeV, since it provides the optimal Ac-225 production yields and purity levels. At 19 MeV, the production yield of Ac-225 is increased by up to 36% when compared to the conventional method described in EP-A-0 962 942.

In practice, the present process is preferably carried out in a cyclotron, which generally permits to accelerate deuterons to the preferred energy range.

The Ra-226 target material preferably is in the form RaCl₂, which has been dried and pressed into pellets.

To facilitate the handling of the highly toxic Ra-226 target material, the latter is advantageously placed in a sealed capsule of aluminium. The capsule provides a leak-free container for the highly toxic Ra-226 and allows target processing after irradiation while preventing introduction of impurities into the medical grade product and avoids the introduction of undesired cations which would interfere with the chelation of the radionuclides.

Before introduction into the capsule, the target material is preferably placed in an envelope made of Ag, Ti or Nb, so as to avoid contamination of the target material with aluminium, in particular during post-irradiation treatments. These metals have a high conductivity and thus allow for a high deuteron current density during irradiation. Nb is particularly preferred for its low ionising radiation emissions after irradiation.

After irradiation, Actinium is preferably chemically separated from the irradiated target of Ra-226.

### BRIEF DESCRIPTION OF THE DRAWINGS

The present invention will now be described, by way of example, with reference to the accompanying drawings, in which:
- FIG.1:: is a graph illustrating the cross-section of reactions Ra-226(d,3n)Ac-225 and Ra-226(p,2n)Ac-225 in function of the incident beam energy;
- FIG.2:: is a graph illustrating the reaction cross-sections corresponding to the different Ac radionuclides in function of the deuteron energy.

### DETAILED DESCRIPTION OF A PREFERRED EMBODIMENT

According to the present method Ac-225 is produced by bombardment of Ra-226 with deuterons. As already explained, Ac-225 results from the transformation of the Ra-226 -due to deuteron bombardment- with emission of three neutrons; this nuclear reaction is noted Ra-226(d,3n)Ac-225. The present method has been found to produce Ac-225 with a higher level of purity and higher yield than the method used up to now based on the reaction of Ra-226 with protons (noted Ra-226(p,2n)Ac-225) as known e.g. from EP-A-0 962 942.

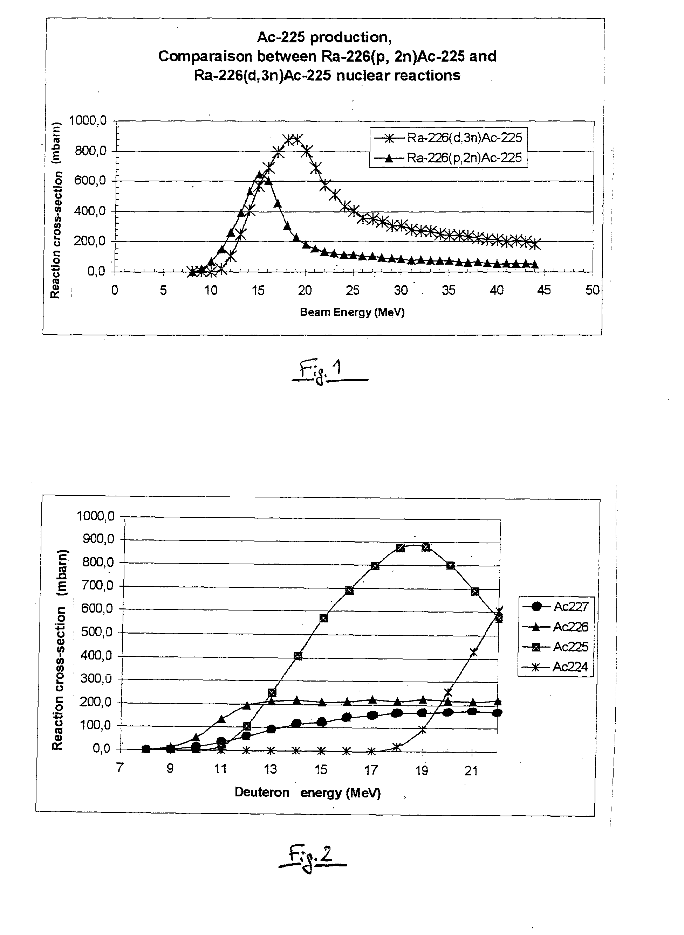

In Fig.1, which was obtained by Monte Carlo simulation, the reaction cross-section for the present reaction (Ra-226(d,3n)Ac-225) and for the known reaction (Ra-226(p,2n)-Ac225) are plotted in function of the energy of the incident particle beam. The cross-section is a numerical quantity (unit: barn) that represents the likelihood that a given atomic nucleus will undergo a specific reaction in relation to a particular species of incident particle. As can be seen, the cross-section of the reaction used in the present method is much higher than that of the conventional reaction (proton bombardment), over a large part of the energy range. It follows that Ac-225 production according to the present method will provide higher yields than the proton irradiation method, in particular in the preferred deuteron energy range of 15 to 22 MeV. The optimal yields will be obtained with a deuteron energy of between 18 and 20 MeV.

The bombardment of Ra-226 with deuterons also leads to the production of radionuclides other than Ac-225, but in lower quantities. In Fig.2, the reaction cross-section of Ra-226 bombardment with deuterons to produce Ac-225 and its isotopes Ac-224, Ac-226 and Ac-227 is plotted versus the deuteron energy. As can be seen, in the preferred energy range of 15 to 22 MeV, the cross-section of Ra-226(d,3n)Ac-225 is largely above the cross-sections of the other Ac radionuclides.

The present method thus provides an improved method of producing Ac-225, which, when implemented with deuterons of adequate energy, provides increased yields and purity levels.

In practice, the present method is preferably implemented as follows. Firstly, Ra-226 target material is prepared, preferably in the form RaCl₂ by precipitation out of concentrated HCl. This target material is then heated to a temperature of about 150 to 200°C to release the crystal water therefrom and pressed into pellets.

Next, the target material, which thus essentially consists of RaCl₂, is introduced into an envelope preferably made of Ag, Ti or Nb. These metals are insoluble in HCl and have a high thermal conductivity, which allows for a high current density during deuteron irradiation. In addition, Nb has the advantage of being less activated by the irradiation than Ag or Ti, thus resulting in a lower ionising radiation emission rate from the envelope.

The Ra-226 target material received in its envelope is then sealed in a capsule made of aluminium. It is to be noted that the previous elimination of crystal water from the RaCl₂ will avoid pressure build up in the capsule. The capsule provides a sealed container for the highly radiotoxic Ra-226, allows target processing after irradiation without introducing impurities into the medical grade product and avoids the introduction of undesired cations that would interfere with the chelation of the radionuclides in the later therapeutic applications.

According to the present method, the Ra-226 target, received in its envelope and in the capsule, is irradiated by a beam of deuterons. The deuteron beam is preferably produced by cyclotron, wherein deuterons can generally be accelerated to the preferred energy range. During irradiation, the capsule is advantageously cooled by a closed water circuit with an alpha monitor to detect any leakage of radon from the capsule. Such a cooling circuit comprises e.g. a pump and a heat exchanger for extracting the heat produced by the irradiation in the capsule.

After irradiation, the target material is dissolved and then treated in a conventional way to separate Ac from Ra, e.g. an ion exchangers.

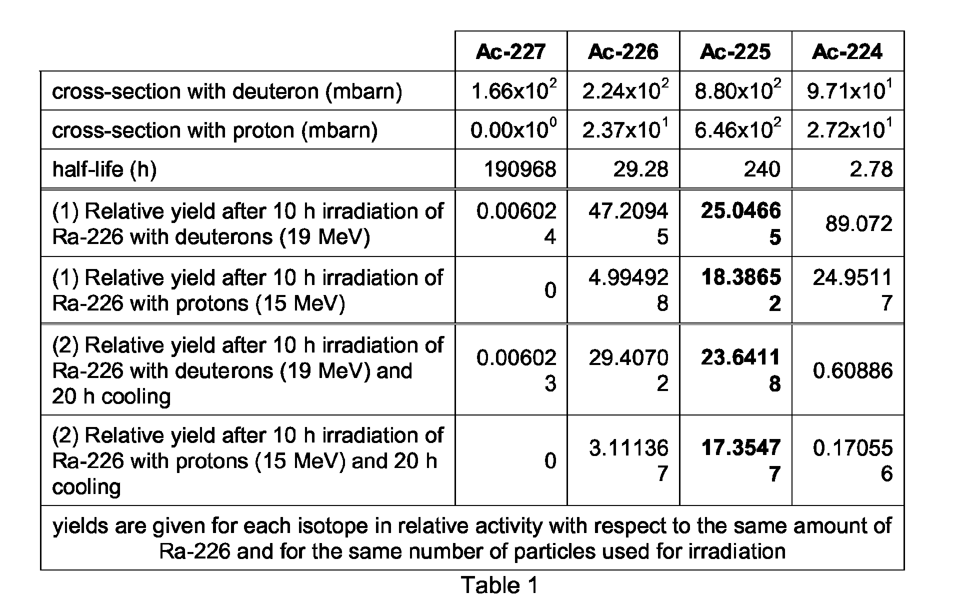

In table 1, calculated relative yields for Ac-225 and its other isotopes Ac-224, Ac-226 and Ac-227 are indicated when produced with the present process or with the conventional process using protons. These values have been calculated for two scenarios: (1) after irradiation with the selected particle beam for 10 hours, and (2) after irradiation with the selected particle beam for 10 hours and 20 hours cooling. As can be seen, the Ac-225 yield obtained by bombarding Ra-226 with a 19 MeV deuterons beam is in both scenarios about 36% higher than the yield obtained with the conventional method.

This table also shows that the long-life Ac-227 is produced in extremely low quantity with respect to Ac-225. Due to their relatively short half-life, the other radionuclides Ac-224 and Ac-225 are rapidly eliminated by natural decay.

## Claims

1. A method for producing Ac-225 **characterised by** irradiating a target of Radium-226 with deuterons.

2. The method according to claim 1, **characterised in that** said deuterons have an incident energy of between 15 and 22 MeV, preferably between 18 and 20 MeV.

3. The method according to claim 1 or 2, **characterised in that** said deuterons are accelerated in a cyclotron.

4. The method according to any one of claims 1 to 3, **characterised in that** said target of Ra-226 is in the form of pellets consisting essentially of radium chloride RaCl₂.

5. The method according to any one of the preceding claims, **characterised in that** during said irradiation, said target is received in a sealed capsule made of aluminium, which is cooled by a closed cooling circuit.

6. The method according to claim 5, **characterised in that** before introduction into said capsule, said target of Ra-226 is placed in an envelope made of Ag, Ti or Nb.

7. The method according to claim 1, **characterised in that** after irradiation, Actinium is chemically separated from the irradiated target of Ra-226.
